(19) Europäisches Patentamt
European Patent Office
Office européen des brevets

(11) Veröffentlichungsnummer: **0 190 708**
**A1**

(12) # EUROPÄISCHE PATENTANMELDUNG

(21) Anmeldenummer: 86101382.9

(22) Anmeldetag: 03.02.86

(51) Int. Cl.⁴: **C07C 93/00** , C07D 295/00

(30) Priorität: 08.02.85 DE 3504242

(43) Veröffentlichungstag der Anmeldung:
**13.08.86 Patentblatt 86/33**

(84) Benannte Vertragsstaaten:
**AT BE CH DE FR GB IT LI NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Lange, Fritz, Dr.**
**Baderweg 82**
**D-4300 Essen(DE)**
Erfinder: **Meffert, Alfred, Dr.**
**Marie-Curie-Strasse 10**
**D-4019 Monheim(DE)**

(54) **Verfahren zur Herstellung von tertiären Etheraminen.**

(57) Verfahren zur Herstellung von tertiären Etheraminan der allgemeinen Formel I

$$R^1 - (OC_nH_{2n})_x(OC_mH_{2m})_y - N \begin{matrix} R^2 \\ R^3 \end{matrix} \qquad (I)$$

durch Umsetzung eines tertiären Amins der Formel II

$$H(OC_mH_{2m})_y - N \begin{matrix} R^2 \\ R^3 \end{matrix} \qquad (II)$$

mit einem Schwefelsäurehalbestersalz der Formel III

$R^1 - (OC_nH_{2n})_x - OSO_3M$ (III)

in Gegenwart von wenigstens 1 Mol einer starken Base. Bevorzugt wird 1 Mol des tertiären Amins der Formel II mit 0,2 - 3,0 Mol des Schwefelsäurehalbestersalzes der Formel III in Gegenwart von 1 - 1,5 Mol einer starken Base aus der Gruppe der Alkalihydroxide oder Alkalialkoholate pro Mol Schwefelsäurehalbestersalz in Abwesenheit von Wasser bei 140° - 230°C zur Umsetzung gebracht.

EP 0 190 708 A1

"Verfahren zur Herstellung von tertiären Etheraminen"

Gegenstand der Erfindung ist ein neues Verfahren zur Herstellung von tertiären Etheraminen, durch welches sowohl an sich bekannte als auch neue Etheramine hergestellt werden können.

Es sind Verfahren zur Herstellung von tertiären Etheraminen bekannt, die ausgehend von primären und sekundären Aminen durch Alkylierung am Stickstoffatom, z.B. mit Alkylpolyglycolethersulfaten zu Etheraminen führen. Solche Verfahren werden z.B. in GB-PS 1,087,413 und in EP-A-102 140 beschrieben. Durch diese Verfahren ist jedoch nur ein begrenztes Spektrum von tertiären Etheraminen zugänglich, da die für diese Verfahren erforderlichen sekundären Amine oft nicht bekannt oder schwer zugänglich sind.

Es wurde nun überraschend gefunden, daß tertiäre Etheramine der allgemeinen Formel I

$$(I)\qquad R^1-(OC_nH_{2n})_x(OC_mH_{2m})_y-N\begin{matrix}R^2\\R^3\end{matrix}$$

in der R' eine Alkyl- oder Alkenylgruppe mit 6 bis 22 C-Atomen oder eine Alkyl- oder Dialkylphenylgruppe mit 6 bis 16 C-Atomen in der Alkylgruppe, n und m Zahlen von 2 bis 4, x = 0 oder eine Zahl von 1 bis 20, y eine Zahl von 1 bis 10, $R^2$ und $R^3$ unabhängig voneinander Alkyl- oder Alkenylgruppen mit 1 bis 22 C-Atomen, Gruppen der Formel H $(OC_mH_{2m})_y$, Gruppen der Formel $R^1(OC_nH_{2n})_x$ - $(OC_mH_{2m})_y$ - oder gemeinsam mit dem Stickstoffatom einen 5- oder 6-gliedrigen Ring bilden, der auch ein weiteres Stickstoff- oder Sauerstoffatom enthalten kann, auf einfache Weise durch Umsetzung eines tertiären Amins der Formel II

$$II\qquad H\ (OC_mH_{2m})y-N\begin{matrix}R^2\\R^3\end{matrix}$$

mit einem Schwefelsäurehalbestersalz der Formel III

III $R^1$- $(OC_nH_{2n})_x$ - $OSO_3$ M

in der M ein Alkali- oder Erdalkaliatom ist, erhalten werden. Die Bedeutung von $R^1$, $R^2$, $R^3$, m, n, x und y in den Formeln II und III ist die gleiche wie für Formel I angegeben.

Wenn nur eine Hydroxylgruppe des tertiären Amins der Formel II mit dem Schwefelsäurehalbestersalz der Formel III alkyliert werden soll, so kann die Reaktion mit äquimolaren Mengen des Schwefelsäurehalbesters (Molverhältnis 1 : 1) durchgeführt werden. Enthält das tertiäre Amin der Formel II noch eine zweite oder dritte Hydroxylgruppe, so empfiehlt sich die Anwendung eines Überschusses des Amins, etwa bis zu 5 Mol/Mol des Schwefelsäurehalbestersalzes, um die Alkylierung einer zweiten Hydroxylgruppe, die als Nebenreaktion abläuft, zu unterdrücken.

Es kann aber auch erwünscht sein, ein tertiäres Amin der Formel II, in der z.B. $R^2$ und gegebenenfalls auch $R^3$ eine Gruppe der Formel H $(OC_mH_{2m})_y$ ist, mit zwei oder drei Mol des Schwefelsäurehalbesters zur Umsetzung zu bringen und auf diese Weise tertiäre Etheramine der Formel I zu erhalten, in welchen $R^2$ oder $R^2$ und $R^3$ Gruppen der Formel $R^1$ $(OC_nH_{2n}O)_x(OC_mH_{2m})_y$ -sind. In diesen Fällen wird 1 Mol des Amins mit 2 oder 3 Mol des Schwefelsäurehalbesters umgesetzt.

Ein Mol des tertiären Amins der Formel II kann daher mit 0,2 bis 3,0 Mol des Schwefelsäurehalbestersalzes der Formel III zur Umsetzung gebracht werden.

Die Umsetzung wird in Gegenwart von wenigstens 1 Mol einer starken Base, bevorzugt eines Alkalihydroxids oder eines Alkalialkoholats, z.B. eines Alkohols mit 1 bis 4 C-Atomen, pro Mol des eingesetzten Schwefelsäurehalbestersalzes, zur Bindung des gebildeten Hydrogensulfats durchgeführt. Dabei wird bei Verwendung von Alkalihydroxid 1 Mol Wasser, bei Verwendung von Alkoholat 1 Mol Alkohol, pro Mol Schwefelsäurehalbestersalz frei, welches während der Umsetzung aus dem Reaktionsgemisch abdestilliert wird. Es empfiehlt sich, die Base in einer Menge von 1 bis 1,5 Mol pro Mol Schwefelsäurehalbestersalz einzusetzen.

Die Umsetzung wird in weitgehender Abwesenheit von Wasser durchgeführt, da dieses unter den Reaktionsbedingungen eine Hydrolyse des Schwefelsäurehalbestersalzes bewirkt.

Für die Umsetzung geeignete wasserfreie Schwefelsäurehalbestersalze der Formel III lassen sich zum Beispiel durch Entwässerung technischer, wässriger Alkylsulfate, Alkylethersulfate oder Alkylphenolpolyglycolethersulfate herstellen.

Ein besonders elegantes Verfahren besteht darin, daß man aus dem Amin der Formel II, einem Schwefelsäurehalbester der Formel IV

(IV) $R^1$- $(OC_nH_{2n})_x$ - $OSO_3H$

und 2 bis 2,5 Mol einer starken Base pro Mol des Schwefelsäurehalbesters ein Reaktionsgemisch herstellt und dieses zur Umsetzung bringt. In Formel IV haben $R^1$, n und x die für Formel I und III angegebene Bedeutung. Diese Arbeitsweise hat den Vorteil, daß Entwässerung des Schwefelsäurehalbestersalzes entfällt und nur das durch Salzbildung und bei der Umsetzung freiwerdende Wasser beziehungsweise der bei Verwendung von Alkoholat als Base gebildete Alkohol aus dem Reaktionsgemisch abdestilliert werden muß.

Die Reaktion läuft bei einer Temperatur von 140 bis 230 °C in einer Zeit von 1 bis 5 Stunden weitgehend quantitativ ab. Die wesentlichen Nebenreaktionen sind die Hydrolyse des Schwefelsäurehalbestersalzes, die Mehrfachalkylierung bei Aminen mit mehreren Hydroxylgruppen und die oxidative Verfärbung. Durch weitgehenden Ausschluß von Wasser, durch Wahl des Molverhältnisses und durch Arbeiten under Schutzgas, z.B. in Stickstoffatmosphäre, lassen sich diese Nebenreaktionen hintanhalten.

Nach beendeter Umsetzung kann des Reaktionsgemisch durch Auswaschen mit Wasser oder, im Falle gut wasserlöslicher Etheramine, mis gesättigter Kochsalzlösung oder Sodalösung aufgearbeitet werden. Auf diese Weise werden die wasserlöslichen Nebenprodukte wie Alkalisulfat, Magnesiumsulfat und nichtumgesetztes Amin aus dem Produkt entfernt. Das Produkt kann dann nach üblichen Verfahren getrocknet werden.

Geeignete tertiäre Amine der Formel II sind z.B. Triethanolamin, Methyldiethanolamin, Dimethylethanolamin, N--Hydroxyethylmorpholin, Dimethylisopropanolamin, Triisopropanolamin, Anlagerungsprodukte von 2 bis 20 Mol Ethylenoxid, Propylenoxid und/oder Butylenoxid an primäre Alkylamine der Formel $R^2$- $NH_2$ oder von 1 bis 10 Mol dieser Alkylenoxide an Dialkylamine der Formel $R^2$- NH- $R^3$, wobei $R^2$ und $R^3$ die vorher genannte Bedeutung haben. Hierzu gehören z. B. die Anlagerungsprodukte von Ethylenoxid oder Propylenoxid an primäre Fettamine, an Dialkylamine oder Morpholin.

Geeignete Schwefelsäurehalbestersalze der allgemeinen Formel III sind z.B. die Lithium-, Natrium-, Kalium- oder Magnesiumsalze der Fettalkoholsulfate mit 6 bis 22 C--Atomen, z.B. das n-Octylsulfat, das n-Laurylsulfat, das n--Cetylsulfat, das n-Stearylsulfat, das Oleylsulfat, das Behenylsulfat oder das n-Erucylsulfat. Geeignet sind aber auch die Scwefelsäurehalbestersalze verzweigter primärer Alkohole mit 6 bis 22 C-Atomen, z.B. das 2-Ethyl-hexylsulfat, das Isononylsulfat oder das 2-Hexyl-decylsulfat. Weitere geeignete Schwefelsäurehalbestersalze der Formel III leiten sich von Anlagerungsprodukten von 1 bis 20 Mol Ethylenoxid, Propylenoxid oder Butylenoxid an primäre und sekundäre Alkohole mit 6 bis 22 C-Atomen oder an Alkylphenol oder Dialkylphenole mit 6 bis 16 C-Atomen in der Alkylgruppe ab. Solche Ethersulfate haben als - schaumstarke Tenside für technische und kosmetische Anwendungen große wirtschaftliche Bedeutung und sind leicht, in großen Mengen und preiswert zugänglich.

Die Anlagerung von Ethylenoxid, Propylenoxid und/oder Butylenoxid an primäre und sekundäre Alkohole und an Alkylphenole wird in Gegenwart von basischen Katalysatoren, z.B. von Natriummethylat, Natriumhydroxid oder Calciumacetat oder von sauren Katalysatoren wie z.B. Bortrifluorid durchgeführt. Dabei werden Homologengemische erhalten, wobei der mittlere Alkoxylierungsgrad dem Molverhältnis des Alkylenoxids zum Alkohol oder Alkylphenol entspricht.

Auch die Anlagerung der Alkylenoxide an primäre und sekundäre Amine, die ebenfalls in Gegenwart von basischen Katalysatoren erfolgt, führt nach der Addition an die NH-Gruppen zum Aufbau von Polyalkylenglycoletherketten, die ein Homologengemisch darstellen, dessen mittlerer Alkoxylierungsgrad dem Molverhältnis des Alkylenoxids zum Amin entspricht. In den allgemeinen Formel I, II, und III stellen die Zahlen x für Werte von 1 bis 20 und y für Werte von 2 bis 10 daher den mittleren Alkoxylierungsgrad des Homologengemisches dar.

Durch die Art und Anzahl der Oxyalkylgruppen einerseits in dem Schwefelsäurehalbestersalz (n und x in Formel III), andererseits in dem tertiären Amin (m und y in Formel II) können die Wasserlöslichkeit und die anwendungstechnischen Eigenschaften der erfindungsgemäß zugänglichen Etheramine in weitem Bereich gesteuert werden. Das erfindungsgemäße Verfahren ermöglicht die Herstellung von Etheraminen, die nach bekannten Verfahren nicht oder nur auf sehr umständlichem Wege zugänglich sind. Die erfindungsgemäß herstellbaren Etheramine sind für zahlreiche Anwendungen geeignet, z.B. als grenzflächenaktive Stoffe und als Ausgangsprodukte zur Herstellung grenzflächenaktiver Folgeprodukte wie z.B. Aminoxide und quartäre Ammoniumverbindungen.

Die folgenden Beispiele sollen den Gegenstand der Erfindung näher erläutern ohne ihn hierauf zu beschränken.

Beispiele

1. n-Octyloxyethyl-dihydroxyethylamin

Zu 100 g Triethanolamin (0,67 Mol) wurden 40,3 g einer 30 %igen (Gew.%) Lösung von Natriummethylat (0,224 Mol) in Methanol gegeben und das Methanol im Vakuum und unter Erwärmung auf 110°C abdestilliert. In die erhaltene Lösung wurden 51,6 g n-Octylsulfat-Na-Salz (0,22 Mol) bei 20 °C eingerührt und das Reaktionsgemisch 4 Stunden auf 200 °C erhitzt. Nach dem Abkühlen auf 20 °C wurden ca. 160 ml Wasser zugegeben. Es bildeten sich zwei flüssige Phasen, von diesen wurde die obere Phase abgetrennt, zweimal mit Wasser gewaschen und unter leichtem Erwärmen auf ca. 80 °C im Vakuum am Rotationsverdampfer getrocknet. Es wurden 52 g einer braunen öligen Flüssigkeit mit einer Aminzahl von 214 erhalten. (Die Aminzahl gibt an, wieviel mg KOH einem Gramm der Substanz äquivalent sind.)

Wenn die Umsetzung unter Stickstoffatmosphäre durchgeführt wurde, konnte das Etheramin mit hellgelber Farbe isoliert werden.

2. Lauryl/myristylpoly(3,6)oxyethyl-dihydroxyethylamin

300 g (0,456 Mol) Fettalkohol $C_{12}/C_{14}$ (1 : 1)-3,6 EO-sulfat--Na-Salz(Schwefelsäurehalbestersalz des Anlagerungsproduktes von 3,6 Mol Ethylenoxid an ein Fettalkoholgemisch aus 50 Gew.% Laurylalkohol und 50 Gew.% Myristylalkohol, 70 Gew,%ige wässrige Lösung) wurden mit 67.9 g (0,456 Mol) Triethanolamin zusammengegeben und im Rotationsverdampfer bei 100 °C im Wasserstrahlvakuum weitgehend entwässert. Nach Spülung des Reaktionsgefässes mit Stickstoff wurden 18,3 g Natriumhydroxid (0,456 Mol) zugesetzt, nochmals mit Stickstoff gespült und das Reaktionsgemisch 3 Stunden auf 200 °C erhitzt. Nach dem Abkühlen auf 20 °C wurde der Ansatz mehrmals mit gesättigter Kochsalzlösung ausgewaschen und am Rotationsverdampfer unter Vakuum und Erwärmen bis 100 °C getrocknet. Ausgefallenes Natriumchlorid wurde abfiltriert. Es wurde ein gelbes Öl in einer Menge von 185,0 g und mit einer Aminzahl von 95 erhalten.

3. Lauryl-myristylpoly(3,6)oxyethyl-hydroxyethyl-methylamin

Nach dem Verfahren analog Beispiel 2 wurden

230 g (0,5 Mol) Fettalkohol $C_{12}C_{14}$(1:1)-3,6 EO-sulfat-Na--Salz (70 %ige; wässrige Lösung)

54,3 g (0,456 Mol) N-Methyl-diethanolamin und

12,8 g Natriumhydroxid

umgesetzt und aufgearbeitet. Es wurde ein hellgelbes Öl in einer Menge von 130 g und mit einer Aminzahl von 104 erhalten.

4. Lauryl-myristylpoly (2) oxyethyl-dihydroxyethylamin

Nach dem Verfahren analog Beispiel 2 wurden

968 g (1,67 Mol) Fettalkohol $C_{12}C_{14}$ (7:3)-2 EO-sulfat Na--Salz (65,8 %ige wässrige Lösung)

300,7 g (2,018 Mol) Triethanolamin und

80,7 g (2,018 Mol) Natriumhydroxid

umgesetzt und aufgearbeitet. Es wurde ein gelbbraunes Öl in einer Menge von 565 g und mit einer Aminzahl von 111,4 erhalten.

5. n-Octyloxyethyl-hydroxyethyl-methylamin

318 g (1,377 Mol) n-Octylsulfat, Na-Salz

491,5 g (4,130 Mol) N-Methyldiethanolamin und

55,1 g (1,377 Mol) Natriumhydroxid

wurde 3 Stunden unter Stickstoffatmosphäre unter Rühren auf 200 °C erhitzt.

Nach dem Abkühlen auf 80 °C wurde der Ansatz viermal mit gesättigter Kochsalzlösung gewaschen und dann im Vakuum bei 80 °C am Rotationsverdampfer getrocknet. Ausgefallenes Natriumchlorid wurde abfiltriert. Es wurden 254 g einer rotbraunen Flüssigkeit mit einer Aminzahl von 238 erhalten.

6. Cetyl-/Stearyl-oxyethyl-dihydroxyethylamin

Nach dem Verfahren analog Beispiel 2 wurden

467 g (0,69 Mol) Cetyl-/Stearyl (30 : 70)-sulfat, Na-Salz - (53,5 %ige wässrige Paste)

149,2 g (1,0 Mol) Triethanolamin

48,0 g (1,2 Mol) Natriumhydroxid

umgesetzt und aufgearbeitet. Es wurde ein dunkles Öl in einer Menge von 238 g und mit einer Aminzahl von 129,6 erhalten.

7. Di(cetyl-/stearyl-oxyethyl)-hydroxyethylamin

338,7 g (0,5 Mol) Cetyl-/Stearyl (30 : 70)-sulfat, Na-Salz - (53,5 %ige wässrige Paste)

201,1 g (0,5 Mol) Cetyl-/Stearyl-oxyethyl--dihydroxyethylamin (gemäß Beispiel 6)

21,0 g (0,53 Mol) Natriumhydroxid

wurden nach dem Verfahren analog Beispiel 2 umgesetzt und aufgearbeitet. Es wurde ein dunkelbraunes Öl in einer Menge von 241 g und mit einer Aminzahl von 70,8 erhalten.

8. Lauryl/myristylpoly (3)oxyethyl-dihydroxyethylamin

404 g (1 Mol) Fettalkohol $C_{12}/C_{14}$ (7 : 3)-3-EO--schwefelsäurehalbester mit einer Temperatur von 40 °C wurden zu 179 g (1,2 Mol) Triethanolamin langsam zugetropft. Dann wurden 84 g (2,1 Mol) Natriumhydroxid zugegeben und das Reaktionsgemisch unter Rühren langsam erwärmt. Ab 145 °C setzte heftiges Aufschäumen ein, und es wurde unter leichtem Vakuum Wasser aus dem Reaktionsgemisch abdestilliert. Das Reaktionsgemisch wurde dann 1,5 Stunden auf 180 °C gehalten und nach dem Abkühlen auf 20 °C wie in Beispiel 2 aufgearbeitet.

Es wurde ein gelbes Öl in einer Menge von 377 g und mit einer Aminzahl von 86,7 erhalten.

9. Lauryloxyethyl-kokos ($C_8$-$C_{18}$-alkyl-hydroxyethylamin

Zu 312 g (1,1 Mol) Kokos($C_8$-$C_{18}$)-alkyl-dihydroxyethylamin (Anlagerungsprodukt von 2 Mol Ethylenoxid an ein Kokosfettamin($C_8$-$C_{18}$) wurden 48 g (1, 2 Mol) Natriumhydroxid und 288,4 g (1 Mol) n-Laurylsulfat-Na-Salz zugegeben und die Mischung in einer Stickstoffatmosphäre langsam erwärmt. Ab 140 °C begann Wasser abzudestillieren. Das Reaktionsgemisch wurde insgesamt 2 Stunden bei 200 °C gehalten. Nach dem Abkühlen auf 20 °C wurde wie in Beispiel 2 aufgearbeitet. Dabei wurden 293 g einer öligen Flüssigkeit mit einer Aminzahl von 137 und einer Hydroxylzahl von 182 erhalten.

10. Lauryloxyethyl-hydroxyethyl-methylamin

Zu 143,1 g (1,2 Mol) N-Methyl-diethanolamin wurden 40 g (1 Mol) Natriumhydroxid und 288,4 g (1 Mol) n-Laurylsulfat--Na-Salz zugegeben und die Mischung analog Beispiel 9 umgesetzt und aufgearbeitet.

Es wurden 156 g eines gelben Öls mit einer Aminzahl von 171 und einer Hydroxylzahl von 153 erhalten.

Durch Destillation wurden aus dem Rohprodukt 95 g (ca. 61 Gew.%) einer bei 125 bis 160 °C/0,001 Torr siedenden Fraktion mit einer Aminzahl von 188 gewonnen.

11. Di-(lauryloxyethyl)-methylamin

43,1 g (0,145 Mol) der gemäß Beispiel 10 gewonnenen Reinfraktion (Aminzahl 188) wurden mit 6,5 g (0,15 Mol) NaOH und 43,3 g (0,15 Mol) Laurylsulfat-Na-Salz analog Beispiel 9 umgesetzt und aufgearbeitet. Es wurden 34 g eines gelben Öls mit einer Aminzahl von 125 erhalten.

12. Lauryloxyethyl-dihydroxyethylamin und Di-(lauryloxyethyl)-hydroxyethylamin

In einen Rührkolben mit Destillationsaufsatz wurden 149,2 g (1 Mol) Triethanolamin, 48 g (1,2 Mol) Natriumhydroxid und 288,4 (1 Mol) Laurylsulfat-Na-Salz zusammen unter Stickstoffatmosphäre langsam erwärmt. Ab 140 °C setzte Wasserabspaltung ein. Das Reaktionsgemisch wurde unter Entfernung von Wasser und flüchtigen Nebenprodukten weiter auf 200 °C erwärmt und bei dieser Temperatur 2 Stunden gehalten. Die Aufarbeitung erfolgte analog Beispiel 9. Es wurden 218 g eines Rohproduktes mit einer Aminzahl von 148 erhalten.

Durch Destillation wurden aus dem Rohprodukt 138 g einer bei 175 °C/0,001 Torr siedenden Fraktion mit einer Aminzahl von 176 gewonnen. Dieses Produkt stellt Lauryloxyethyl-dihydroxyethylamin dar (theor. Aminzahl 176,3).

Weiterhin wurden 67 g einer zweiten, bei 235 °C/0,001 Torr siedenden Fraktion mit einer Aminzahl von 118 gewonnen. Dieses Produkt entspricht dem Di-(lauryloxyethyl)-hydroxyethylamin (theor. Aminzahl 115,7).

Außer den genannten Fraktionen wurden 10 g Vorlauf und 3 g Rückstand erhalten.

13. Tris (lauryloxyethyl)-amin

20 g (0,042 Mol) Di (lauryloxyethyl)-hydroxyethylamin - (zweite Fraktion nach Beispiel 12) wurden mit 2,0 g (0,05 Mol) Natriumhydroxid und 12,1 g (0,042 Mol) Laurylsulfat-Na-Salz analog Beispiel 12 umgesetzt und aufgearbeitet. Es wurden 32 g eines gelben öligen Produktes mit einer Aminzahl von 85,6 erhalten.

14. Lauryloxyethyl-diethylamin

140,6 g (1,2 Mol) Diethylethanolamin, 48 g (1,2 Mol) Natriumhydroxid und 288,4 g (1 Mol) Laurylsulfat-Na-Salz wurden unter Stickstoffatmosphäre analog Beispiel 9 umgesetzt. Während der Reaktion destillierten mit dem Wasser ca. 20 g des Diethylethanolamins ab, die sogleich wieder ersetzt wurden. Nach 2 Stunden Reaktionszeit bei 190 °C und Abkühlung auf 20 °C wurde analog Beispiel 2 aufgearbeitet.

Es wurden 168 g einer öligen Flüssigkeit mit einer Aminzahl von 151 erhalten.

15. N-Lauryloxyethyl-morpholin

157,5 g (1,2 Mol) N-2-Hydroxyethyl-morpholin, 48 g (1,2 Mol) Natriumhydroxid und 288,4 g (1 Mol) Laurylsulfat-Na-Salz wurden unter Stickstoffatmosphäre analog Beispiel 9 umgesetzt und aufgearbeitet. Es wurden 246 g Rohprodukt erhalten, aus welchem nach mehrfachem Waschen mit Kochsalzlösung 59 g einer öligen Flüssigkeit mit einer Aminzahl von 154 isoliert werden konnten.

**Ansprüche**

1. Verfahren zur Herstellung von tertiären Etheraminen der allgemeinen Formel I,

$$\text{(I)} \quad R^1(OC_nH_{2n})_x(OC_mH_{2m})_y - N{<}^{R^2}_{R^3}$$

in der $R^1$ eine Alkyl- oder Alkenylgruppe mit 6 bis 22 C-Atomen oder eine Alkyl- oder Dialkylphenylgruppe mit 6 bis 16 C-Atomen in der Alkylgruppe, n und m Zahlen von 2 bis 4, x = 0 oder eine Zahl von 1 bis 20, y eine Zahl von 1 bis 10, $R^2$ und $R^3$ unabhängig voneinander Alkyl- oder Alkenylgruppen mit 1 bis 22 C-Atomen, Gruppen der Formel $H(OC_mH_{2m})_y$, Gruppen der Formel $R^1$-$(OC_nH_{2n})_x$-$(OC_mH_{2m})_y$ - oder gemeinsam mit dem Stickstoffatom einen 5- oder 6-gliedrigen Ring bilden, der ein weiteres Stickstoff- oder Sauerstoffatom enthalten kann, dadurch gekennzeichnet, daß man ein tertiäres Amin der Formel II

$$\text{II} \quad H(OC_mH_{2m})_y - N{<}^{R^2}_{R^3}$$

mit einem Schwefelsäurehalbestersalz der Formel III

III $R^1$- $(OC_nH_{2n})_x$ - $OSO_3M$

in der M ein Alkali- oder Erdalkaliion ist, in Gegenwart von wenigstens 1 Mol einer starken Base zur Umsetzung bringt.

2. Verfahren nach Anspruch 1, dadurch gekennzeichnet, daß man 1 Mol des tertiären Amins der Formel II mit 0,2 bis 3,0 Mol des Schwefelsäurehalbestersalzes der Formel III in Gegenwart von 1 bis 1,5 Mol einer starken Base aus der Gruppe der Alkalihydroxide oder Alkalialkoholate pro Mol Schwefelsäurehalbestersalz in Abwesenheit von Wasser bei einer Temperatur von 140 bis 230 °C zur Umsetzung bringt.

3. Verfahren nach Anspruch 1 oder 2, dadurch gekennzeichnet, daß man aus dem tertiären Amin der Formel II, einem Schwefelsäurehalbester der Formel IV

(IV) $R^1$ - $(OC_nH_{2n})_x$ $OSO_3H$

und 2 bis 2,5 Mol der starken Base pro Mol des Schwefelsäurehalbesters ein Reaktionsgemisch herstellt und dieses zur Umsetzung bringt.

Europäisches Patentamt

## EUROPÄISCHER RECHERCHENBERICHT

Nummer der Anmeldung

EP 86 10 1382

<table>
<tr><th colspan="3">EINSCHLÄGIGE DOKUMENTE</th><th></th></tr>
<tr><th>Kategorie</th><th>Kennzeichnung des Dokuments mit Angabe, soweit erforderlich, der maßgeblichen Teile</th><th>Betrifft Anspruch</th><th>KLASSIFIKATION DER ANMELDUNG (Int Cl.4)</th></tr>
<tr><td>X</td><td>DE-A-2 842 217 (BASF AG)<br>* Ansprüche; Beispiel 1 *<br>---</td><td>1-3</td><td rowspan="4">C 07 C 93/00<br>C 07 D 295/00</td></tr>
<tr><td>X</td><td>GB-A-2 045 237 (UNION CARBIDE)<br>* Seite 1, Zeile 80 - Seite 2, Zeile 5 *<br>---</td><td>1-3</td></tr>
<tr><td>X</td><td>GB-A-1 070 307 (CHAS, PFIZER & CO., INC.)<br>* Beispiele *<br>---</td><td>1-3</td></tr>
<tr><td>A</td><td>GB-A-1 087 413 (MARCHON PRODUCTS)<br>* Seite 2, Zeilen 1-20; Beispiele *<br>-----</td><td></td></tr>
<tr><td></td><td></td><td></td><td>RECHERCHIERTE SACHGEBIETE (Int. Cl.4)<br><br>C 07 C 93/00<br>C 07 D 295/00</td></tr>
</table>

Der vorliegende Recherchenbericht wurde für alle Patentansprüche erstellt.

| Recherchenort | Abschlußdatum der Recherche | Prüfer |
|---|---|---|
| DEN HAAG | 16-05-1986 | PAUWELS G.R.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument